# EUROPEAN PATENT APPLICATION

(11) **EP 4 342 914 A1**
(43) Date of publication of application: **27.03.2024**
(21) Application number: 21940919.0
(22) Date of filing: 01.06.2021
(51) Int. Cl.: C07K 16/28, A61P 35/00, A61K 39/00

(54) **ANTI-BCAM ANTIBODY OR ANTIGEN-BINDING FRAGMENT THEREOF**

(30) Priority: 21.05.2021 KR 20210065517
(71) Applicant: Genome and Company, Seongnam-si, Gyeonggi-do 13486 (KR)
(72) Inventor: CHA, Mi Young, Seongnam-si, Gyeonggi-do 13486 (KR); YU, Hyunkyung, Seongnam-si, Gyeonggi-do 13486 (KR); JEON, Bu Nam, Seongnam-si, Gyeonggi-do 13486 (KR); KIM, Hyunuk, Seongnam-si, Gyeonggi-do 13486 (KR)
(74) Representative: Dehns
(86) International application number: PCT/KR2021/006816
(87) International publication number: WO 2022/244908

(57) **Abstract**

The present invention relates to an anti-BCAM antibody or an antigen-binding fragment thereof, and uses thereof. In addition, the anti-BCAM antibody or antigen-binding fragment thereof according to the present invention can be used for preventing or treating a state associated with cells expressing BCAM, for example, cancer..

## Description

### Technical Field

The present invention relates to an anti-BCAM antibody or an antigen-binding fragment thereof, and a use thereof. In addition, the anti-BCAM antibody or antigen-binding fragment thereof of the present invention may be used for conditions associated with cells expressing BCAM, such as for preventing or treating cancer.

### Background Art

A basal cell adhesion molecule (BCAM) is a Ig superfamily transmembrane protein, and is also known as Lutheran blood group glycoprotein (Lu). Lu was initially studied as an antigen of the Lutheran blood type system, and BCAM was identified as an up-regulated antigen in ovarian cancer. Lu and BCAM have the same extracellular domain, but the cytoplasmic tail is different. Specifically, BCAM lacks 40 amino acids at the COOH-terminus present in the Lu cytoplasmic tail. Furthermore, the Lu-specific cytoplasmic region has SH3 binding motifs, dileucine motifs, and potential phosphorylation sites. The shared regions of the Lu and BCAM cytoplasmic tails contain spectrin binding motifs. Since the structures of BCAM and Lu overlap each other, it is difficult to distinguish between Lu and BCAM in actual tissue, and Lu and BCAM are interchangeably referred, referred to as Lu/BCAM, or also referred to as CD239.

The extracellular domain of BCAM includes a domain of one V- set, one C1- set, and three I- sets (V-C1-I-I-I). Since BCAM specifically binds to laminin α5, which is a major component of the basal membrane, BCAM is believed to be involved in cell adhesion to the basal membrane. Laminin α5 binds to the β and γ chains to form a heterotrimer, which is found in many basal membranes of normal tissue and disease tissue. In addition, BCAM promotes migration of lung cancer cells on laminin-511 (LM-511) composed of α5, β1, and γ1 chains. Further, the migration of tumor cells on LM-511 is inhibited in the presence of functional inhibitory antibodies against BCAM. It was confirmed that the overexpression of BCAM was observed not only in ovarian carcinoma but also in skin cancer and hepatocellular carcinoma, and accordingly, BCAM has been proposed as an antigen useful for the diagnosis and development of antibody drugs.

### [Prior Art Document]

### [Non-Patent Document]

Yamato Kikkawa et al., Scientific Report, 2018 Apr 26;8(1):6612

### Disclosure of the Invention

### Technical Problem

The purpose of the present invention is to provide an anti-BCAM antibody or an antigen-binding fragment thereof, which specifically binds to a BCAM protein.

In addition, the present invention provides a use of the antibodies or antigen-binding fragments for preventing or treating conditions associated with cells expressing BCAM, such as cancer.

### Solution to Problem

The present invention provides an anti-BCAM antibody or an antigen-binding fragment thereof.

The present invention provides a pharmaceutical composition for preventing or treating cancer, the pharmaceutical composition comprising the anti-BCAM antibody or antigen-binding fragment thereof. In addition, the present invention provides an antibody or antigen-binding fragment in the form of an antibody-drug conjugate (ADC) capable of selectively delivering an inhibitor in order to target BCAM-expressing cells.

In an embodiment, the present invention provides an anti-BCAM antibody or an antigen binding fragment thereof comprising a heavy chain CDR1 comprising an amino acid sequence of any one selected from the group consisting of SEQ ID NOs: 7, 9 and 11, a heavy chain CDR2 comprising an amino acid sequence of any one selected from the group consisting of SEQ ID NOs: 13, 15 and 17, a heavy chain CDR3 comprising an amino acid sequence of any one selected from the group consisting of SEQ ID NOs: 19, 21 and 23, a light chain CDR1 comprising an amino acid sequence of any one selected from the group consisting of SEQ ID NOs: 8, 10 and 12, a light chain CDR2 comprising an amino acid sequence of any one selected from the group consisting of SEQ ID NOs: 14, 16 and 18, and a light chain CDR3 comprising an amino acid sequence of any one selected from the group consisting of SEQ ID NOs: 20, 22 and 24.

In an embodiment, the present invention provides an anti-BCAM antibody or antigen-binding fragment thereof comprising a heavy chain variable region of any one selected from the group consisting of SEQ ID NOs: 1, 3 and 5; and a light chain variable region of any one selected from the group consisting of SEQ ID NOs: 2, 4 and 6.

In an embodiment, the antigen-binding fragment of the present invention may be Fab, Fab', Fab'-SH, Fv, a single chain antibody scFv, a F(ab')2 fragment, VL, VH, diabody, triabody, tetrabody, minibody (scFV(CH3)2), IgG deltaCH2, scFv-Fc, (scFv)2-Fc, Fynomer, dual-affinity re-targeting (DART), or TRIDENT, which comprises the CDR sequences of the present invention. The antibody of the present invention may be a chimeric antibody, a humanized antibody, or a human antibody, and may be a multi-specific antibody.

In another embodiment, the present invention provides a nucleic acid molecule encoding the antibody or antigen-binding fragment thereof and a recombinant expression vector including the nucleic acid molecule.

The present invention also provides a composition for preventing or treating cancer, the composition comprising the antibody or antigen-binding fragment thereof as an active ingredient. The pharmaceutical composition may be used in combination with an additional anticancer agent, such as another anticancer agent or a chemotherapeutic agent, or may be used in combination with radiation therapy.

The present invention also provides a composition for analyzing or detecting a BCAM protein, the composition comprising the antibody or antigen binding fragment thereof.

### Advantageous Effects of Invention

The anti-BCAM antibody or antigen-binding fragment thereof of the present invention binds to a human BCAM protein with high affinity. As a result, the anti-BCAM antibody or antigen-binding fragment thereof of the present invention can be effectively used to prevent or treat cancer.

### Brief Description of Drawings

FIGS. 1a and 1b show amino acid sequences of a heavy chain variable region (1a) and a light chain variable region (1b) of the selected antibody. The bold and underlined sequences represent CDR sequences of each region.
FIG. 2 shows the binding ability of the antibody of the present invention to BCAM according to ELISA analysis.
FIG. 3 shows the binding ability of the antibody of the present invention to BCAM according to FACS analysis (PBS and human IgG values are indicated by being overlapped).
FIGS. 4a, 4b and 4c show the binding ability of the antibody of the present invention to BCAM according to Octet analysis.
FIG. 5 shows the degree of cellular internalization of the antibody of the present invention observed through a confocal laser scanning microscope.

### Best Mode for Carrying out the Invention

### Anti-BCAM Antibody or Antigen-binding Fragment Thereof

The present invention provides an anti-BCAM antibody or an antigen-binding fragment thereof, which specifically binds to a BCAM protein.

In an embodiment, the anti-BCAM antibody or antigen binding fragment thereof of the present invention comprises:
a heavy chain CDR1 comprising an amino acid sequence of any one selected from the group consisting of SEQ ID NOs: 7, 9 and 11,
a heavy chain CDR2 comprising an amino acid sequence of any one selected from the group consisting of SEQ ID NOs: 13, 15 and 17,
a heavy chain CDR3 comprising an amino acid sequence of any one selected from the group consisting of SEQ ID NOs: 19, 21 and 23,
a light chain CDR1 comprising an amino acid sequence of any one selected from the group consisting of SEQ ID NOs: 8, 10 and 12,
a light chain CDR2 comprising an amino acid sequence of any one selected from the group consisting of SEQ ID NOs: 14, 16 and 18, and
a light chain CDR3 comprising an amino acid sequence of any one selected from the group consisting of SEQ ID NOs: 20, 22 and 24.

In a specific embodiment, the anti-BCAM antibody or antigen binding fragment thereof of the present invention comprises:
(a) a heavy chain CDR1 comprising the amino acid sequence of SEQ ID NO: 7;
   a heavy chain CDR2 comprising the amino acid sequence of SEQ ID NO: 13;
   a heavy chain CDR3 comprising the amino acid sequence of SEQ ID NO: 19;
   a light chain CDR1 comprising the amino acid sequence of SEQ ID NO: 8;
   a light chain CDR2 comprising the amino acid sequence of SEQ ID NO: 14; and
   a light chain CDR3 comprising the amino acid sequence of SEQ ID NO: 20;
(b) a heavy chain CDR1 comprising the amino acid sequence of SEQ ID NO: 9;
   a heavy chain CDR2 comprising the amino acid sequence of SEQ ID NO: 15;
   a heavy chain CDR3 comprising the amino acid sequence of SEQ ID NO: 21;
   a light chain CDR1 comprising the amino acid sequence of SEQ ID NO: 10;
   a light chain CDR2 comprising the amino acid sequence of SEQ ID NO: 16; and
   a light chain CDR3 comprising the amino acid sequence of SEQ ID NO: 22; or
(c) a heavy chain CDR3 comprising the amino acid sequence of SEQ ID NO: 11;
   a heavy chain CDR2 comprising the amino acid sequence of SEQ ID NO: 17;
   a heavy chain CDR3 comprising the amino acid sequence of SEQ ID NO: 23;
   a light chain CDR1 comprising the amino acid sequence of SEQ ID NO: 12;
   a light chain CDR2 comprising the amino acid sequence of SEQ ID NO: 18; and
   a light chain CDR3 comprising the amino acid sequence of SEQ ID NO: 24.

As used herein, that each chain or a variable region thereof comprises a specific amino acid sequence means that the chain or variable region comprises the entire amino acid sequence, has the entire amino acid sequence, or consists of the amino acid sequence.

In one example, the antibody or antigen-binding fragment thereof comprises: a heavy chain CDR1 having the amino acid sequence of SEQ ID NO: 7; a heavy chain CDR2 having the amino acid sequence of SEQ ID NO: 13; a heavy chain CDR3 having the amino acid sequence of SEQ ID NO: 19; a light chain CDR1 having the amino acid sequence of SEQ ID NO: 8; a light chain CDR2 having the amino acid sequence of SEQ ID NO: 14; and a light chain CDR3 having the amino acid sequence of SEQ ID NO: 20.

In another example, the antibody or antigen binding fragment thereof comprises: a heavy chain CDR1 consisting essentially of the amino acid sequence of SEQ ID NO: 7; a heavy chain CDR2 consisting essentially of the amino acid sequence of SEQ ID NO: 13; a heavy chain CDR3 consisting essentially of the amino acid sequence of SEQ ID NO: 19; a light chain CDR1 consisting essentially of the amino acid sequence of SEQ ID NO: 8; a light chain CDR2 consisting essentially of the amino acid sequence of SEQ ID NO: 14; and a light chain CDR3 consisting essentially of the amino acid sequence of SEQ ID NO: 20.

In another example, the antibody or antigen binding fragment thereof comprises: a heavy chain CDR1 consisting of the amino acid sequence of SEQ ID NO: 7; a heavy chain CDR2 consisting of the amino acid sequence of SEQ ID NO: 13; a heavy chain CDR3 consisting of the amino acid sequence of SEQ ID NO: 19; a light chain CDR1 consisting of the amino acid sequence of SEQ ID NO: 8; a light chain CDR2 consisting of the amino acid sequence of SEQ ID NO: 14; and a light chain CDR3 consisting of the amino acid sequence of SEQ ID NO: 20.

As used herein, the terms "comprising," "comprise," or modified words thereof refer to an open meaning. As an example, an antibody or antigen-binding fragment thereof comprising the amino acid sequence listed may include additional amino acid sequences not listed, whether essential or not.

As used herein, the term "consisting essentially of" or a modified phrase thereof includes any of the mentioned elements, and permits the presence of elements that do not substantially affect the basic and novel or functional characteristics of the embodiment. As an example, an antibody or antigen-binding fragment thereof consisting essentially of the amino acid sequence listed may include a substitution of one or more amino acid residues that do not substantially affect the characteristics of the antibody or fragment thereof.

As used herein, the term "consisting of" or a modified phrase thereof refers to the case where respective components as described herein do not permit any element that is not recited or listed in that description of the embodiment.

Each chain or a variant region of another antibody or antigen-binding fragment thereof as defined herein may also include, have, or consist of an amino acid sequence as in the above examples.

The term "complementarity determining region" (CDR; CDR1, CDR2, and CDR3) refers to the amino acid residues within the antibody variable regions, which are necessary for antigen binding. Each variable region typically has three CDR regions identified as CDR1, CDR2, and CDR3.

In another embodiment, the anti-BCAM antibody or antigen-binding fragment thereof of the present invention comprises:
a heavy chain variable region of any one selected from the group consisting of SEQ ID NOs: 1, 3 and 5; and
a light chain variable region of any one selected from the group consisting of SEQ ID NOs: 2, 4 and 6.

In a specific embodiment, the anti-BCAM antibody or antigen-binding fragment thereof of the present invention comprises:
(a) a heavy chain variable region comprising an amino acid sequence of SEQ ID NO: 1 and a light chain variable region comprising an amino acid sequence of SEQ ID NO: 2;
(b) a heavy chain variable region including an amino acid sequence of SEQ ID NO: 3 and a light chain variable region comprising an amino acid sequence of SEQ ID NO: 4; or
(c) a heavy chain variable region comprising an amino acid sequence of SEQ ID NO: 5 and a light chain variable region comprising an amino acid sequence of SEQ ID NO: 6.

The antibody variable regions refer to light and heavy chain portions of an antibody molecule comprising the amino acid sequences of CDRs and framework regions (FR).

The term "antibody" as used herein refers to an immunoglobulin molecule capable of specifically binding to a target, such as a carbohydrate, polynucleotide, lipid, polypeptide, protein, etc., through at least one antigen recognition site, located in the variable region of the immunoglobulin molecule. The term "antibody" herein is used in the broadest sense, and thus broadly encompasses not only intact polyclonal or monoclonal antibodies, but also dimers, multimers, multi-specific antibodies (e.g., bispecific antibodies), antigen-binding fragments thereof, antibody fragments thereof, fusion proteins including any other modified configuration of the immunoglobulin molecule that includes an antigen recognition site (e.g., variable region), synthetic antibodies (e.g., "antibody mimetics"), "FynomAb," and the like.

An antibody includes five classes of immunoglobulin (Ig)M, IgD, IgG, IgA, and IgE, comprising heavy chains made from the heavy chain constant region genes µ, δ, γ, α, and ε, respectively. The light chain and the heavy chain of the antibody are divided into variable regions having different amino acid sequences for each antibody and constant regions having the same amino acid sequence, and the heavy chain constant regions comprise CH1, H (hinge), CH2, and CH3 domains. Each domain consists of two β-sheets and an intramolecular disulfide bond is linked therebetween.

Herein, an antibody comprising a heavy chain variable region comprising the amino acid sequence of SEQ ID NO: 1 and a light chain variable region comprising the amino acid sequence of SEQ ID NO: 2 is referred to as "111a," an antibody comprising a heavy chain variable region comprising the amino acid sequence of SEQ ID NO: 3 and a light chain variable region comprising the amino acid sequence of SEQ ID NO: 4 is referred to as "111b," and an antibody comprising a heavy chain variable region comprising the amino acid sequence of SEQ ID NO: 5 and a light chain variable region comprising the amino acid sequence of SEQ ID NO: 6 is referred to as "111c."

The antibody of the present invention may be a chimeric antibody, a humanized antibody, or a human antibody.

As used herein, the term "chimeric antibody" refers to antibodies in which the variable region sequences are derived from one species and the constant region sequences are derived from another species, such as an antibody in which the variable region sequences are derived from a mouse antibody and the constant region sequences are derived from a human antibody. Methods for producing chimeric antibodies are known in the art. See, for example, Morrison, Science 229:1202 (1985), and the like, which are incorporated herein by reference in its entirety.

As used herein, the term "humanized antibody" refers to antibodies in which one or more CDR sequences derived from the germline of a non-human species, such as another mammalian species, e.g., a mouse or chicken, have been grafted onto framework sequences from a human immunoglobulin molecule. The framework sequences may further be remodified via mutation, for example. Human Ig sequences may be disclosed in, for example, NCBI Database (Entez Gene), etc., by reference. Suitable sequences may be used to reduce immunogenicity of the antibody, or reduce, enhance or modify binding, affinity, on-rate, off-rate, avidity, specificity, half-life, or any other suitable characteristic.

As used herein, the term "human antibody" refers to antibodies in which both frameworks and CDR regions comprise variable regions derived from human immunoglobulin sequences. The constant regions of antibodies are also derived from human immunoglobulin sequences.

As used herein, the term "antigen binding fragment" or "antibody fragment" typically refers to comprising at least a portion (e.g. one or more CDRs) of the antigen binding domain or variable regions of at least the parental antibody. An antibody fragment retains at least some of the binding specificity of the parent antibody. Examples of antigen-binding fragments that can be used herein include, but are not limited to, Fab, Fab', Fab'-SH, Fv, a single chain antibody scFv, a F(ab')2 fragment, VL, VH, diabodies, triabodies, tetrabodies, minibodies ((scFV-CH3)2), IgG deltaCH2, scFv-Fc, (scFv)2-Fc, Fynomer, a dual affinity re-targeting (DART), anticalins, FN3 monobodies, DARPins, Affibodies, Affilins, Affimers, Affitins, Alphabodies, Avimers, Im7, VLR, VNAR, Trimab, CrossMab, TRIDENT, nanobodies, binobodies or di-sdFv.

Specifically, the Fab fragment refers to a monovalent fragment composed of VL, VH, CL, and CH1 domains.

The Fab' fragment differs from the Fab fragment in that several residues are added at the carboxyl terminus of the CH1 domain comprising at least one cysteine from the antibody hinge region.

The Fab'-SH refers to a Fab' in which the cysteine residue of the constant domain has a free thiol group.

The F(ab')2 antibody fragment is generated as a pair of Fab' fragments via a hinge cysteine between the Fab' fragments.

The Fv is a minimum antibody fragment containing a complete antigen-recognition site and -binding site. This fragment consists of a dimer of one heavy chain variable region and one light chain variable region in tight, non-covalent association. From the folding of these two regions emanate six hypervariable loops (three loops each from the heavy and light chain) that contribute the amino acid residues for antigen-binding and confer antigen binding specificity to the antibody. However, even a single variable region has the ability to recognize and bind antigen, although at a lower affinity than the entire binding site.

The single chain antibody scFv is an antibody fragment that comprises VH and VL antibody domains linked into a single polypeptide chain. Preferably, the scFv polypeptide further comprises a polypeptide linker between the VH and VL domains that enables the scFv to form the desired structure for antigen binding. The scFv polypeptide herein is also referred to scFv antibody fragment, antigen-binding fragment scFv, scFv antibody, antibody scFv, or simply scFv.

The diabodies refer to small antibody fragments prepared by constructing scFv fragments using short linkers (about 5-10 residues) between the VH and VL domains such that inter-chain but not intra-chain pairing of the V domains is achieved, resulting in a bivalent fragment, i.e., a fragment having two antigen-binding sites. Bispecific diabodies are heterodimers consisting of two "crossover" scFv fragments in which the VH and VL domains of the two antibodies are present on different polypeptide chains. Similarly, the triabody and the tetrabody comprise three polypeptide chains and four polypeptide chains, respectively, and form three antigen binding sites and four antigen binding sites, respectively, which may be the same as or different from each other.

The Fynomer refers to a non-immunoglobulin-derived binding polypeptide derived from a human Fyn SH3 domain. The Fyn SH3-derived polypeptides are well known in the art and are, for example, disclosed in Grabulovski et al. (2007) JBC, 282, p. 3196-3204, WO 2008/022759, and the like. The Fynomer may be genetically fused with another molecule (e.g., antibody) to produce a form that may be engineered to have "FynomAb," i.e., dual specificity.

Dual-affinity re-targeting (DART) and TRIDENT refer to those designed to bind to two or more targets simultaneously. DART refers to a dual specific diabody covalently linked, such as a diabody linked via a C-terminal disulfide bridge, and its structure and definition, etc. are disclosed in J. Mol. Biol. (2010) 399, 436-449, etc.

The antibody or antigen-binding fragment thereof of the present invention may specifically bind to a BCAM protein, such as a human BCAM protein.

In one specific embodiment, the present invention provides an anti-BCAM antibody or antigen-binding fragment thereof that specifically binds to human BCAM, which binds to the same epitope as an epitope to which the antibody 111a, 111b or 111c binds. The CDR1, CDR2, and CDR3 of each of the heavy and light chains of the antibody or antigen binding fragment thereof have at least 80%, preferably at least 90%, and particularly preferably 100% homology with the CDR1, CDR2, and CDR3 of each of the heavy and light chains of the antibody 111a, 111b or 111c. Similarly, each of the heavy and light chains of the antibody or antigen binding fragment thereof has at least 80%, preferably at least 90%, and particularly preferably 100% homology with each of the heavy and light chains of the antibody 111a, 111b or 111c.

As used herein, the term "BCAM" refers to a basal cell adhesion molecule, which is a surface glycoprotein acting as a receptor for the extracellular matrix protein, laminin. "BCAM" is also known as Lutheran blood group glycoprotein (Lu) or CD239, and herein "BCAM," "Lu," "Lu/BCAM," and "CD239" refer to the same protein and may be used interchangeably.

As used herein, the term "specifically binds to" or "specific for" refers to measurable and reproducible interactions such as binding between a target and an antibody, which is determinative of the presence of the target in the presence of a heterogeneous population of molecules including biological molecules. For example, an antibody that specifically binds to a specific target (e.g., an epitope) is an antibody that binds this target with greater affinity, avidity, more readily, and/or with greater duration than it binds to other targets.

As used herein, the term "specifically binding to a human BCAM protein" may refer to an antibody that binds to a human BCAM protein with a binding dissociation equilibrium constant (K_{D}) of 1 × 10⁻⁷ M or less, or preferably 5 × 10⁻⁸ M or less. Accordingly, the anti-BCAM antibody or antigen binding fragment thereof of the present invention may bind to a human BCAM protein with a binding dissociation equilibrium constant (K_{D}) of 1 × 10⁻⁷ M or less, preferably, a K_{D} of 5 × 10⁻⁸ M or less.

As used herein, the term "K_{D}" refers to a binding dissociation equilibrium constant of a particular antibody-antigen interaction, and is calculated from the equation K_{D} = K_{d}/Kₐ (where Kₐ is the binding rate constant and Kd is the dissociation rate constant), and the constant K_{D} has a unit of M. The K_{D} values for antibodies can be determined using methods widely established in the art. A preferred method for measuring the K_{D} value of an antibody is by using surface plasmon resonance (SRP), preferably a biosensor system such as a Biacore^{®} system, or using a Bio-layer Interferometry (BLI) such as an Octet^{®} system. In one specific example, the K_{D} described herein may be a value obtained through the BLI using the Octet^{®} system.

In another embodiment, the antibody or antigen-binding fragment thereof of the present invention may form a drug-conjugated form, i.e., an antibody-drug conjugate (ADC). As used herein, the term "antibody-drug conjugate" or "ADC" may be represented by the formula M-[L-D]ₙ, wherein M represents an antibody molecule, i.e., an anti-BCAM antibody of the present invention or an antigen-binding fragment thereof, L is an optional linker or linker unit, D is a suitable drug or prodrug, and n is an integer from about 1 to about 20. The drug included in the ADC may be appropriately selected according to therapeutic or diagnostic use, as long as the drug does not interfere with specific binding of the antibody of the present invention. In an embodiment, the drug includes, but is not limited to, a cytotoxic agent (e.g., a chemotherapeutic agent), a prodrug converting enzyme, a radioactive isotope or compound, or a toxin. Drugs and linkers that may be included in the ADC and preparation methods thereof may be according to methods known in the art. Accordingly, the present invention provides an antibody-drug conjugate comprising an anti-BCAM antibody or an antigen-binding fragment thereof.

In another embodiment, the anti-BCAM antibody or antigen binding fragment thereof of the present invention binds to the BCAM protein with an EC50 of 150 nM or less, such as 130 nM or less, 100 nM or less, 50 nM or less, 20 nM or less, or 10 nM or less as determined by ELISA assay.

As used herein, the term "EC50" is a term related to an in vitro or in vivo assay using an antibody, which refers to the concentration of an antibody that induces 50% of the maximum response, i.e., an intermediate response between the maximum response and baseline.

### Nucleic Acid Molecule and Vector

Another aspect of the present invention relates to a nucleic acid molecule encoding the anti-BCAM antibody or antigen-binding fragment thereof of the present invention.

The nucleic acid may be present in whole cells, in a cell lysate, or in a specifically purified or substantially pure form. A nucleic acid is "isolated" or "rendered substantially pure" when purified away from other cellular components or other contaminants, e.g., other cellular nucleic acids or proteins, by standard techniques, including alkaline/SDS treatment, CsCl banding, column chromatography, agarose gel electrophoresis and others well known in the art.

The nucleic acid of the present invention may be, for example, DNA or RNA and may or may not contain intronic sequences. In a preferred embodiment, the nucleic acid is a cDNA molecule.

In an embodiment, the nucleic acid molecule of the present invention encodes the light chain region, the heavy chain region, or both the light chain and the heavy chain regions of the anti-BCAM antibody or antigen-binding fragment thereof of the present invention, and preferably encodes the light chain variable region, the heavy chain variable region, or both the light chain and the heavy chain variable regions.

Once DNA fragments encoding the VL and/or VH regions are obtained, such DNA fragments can be further manipulated by standard recombinant DNA techniques, for example, to thus convert the variable region genes to full-length antibody chain genes, to Fab fragment genes or to a scFv gene. In these manipulations, a VL- or VH-encoding DNA fragment is operatively linked to another DNA fragment encoding another protein, for example, an antibody constant region or a flexible linker. As used herein, the term "operatively linked" means that the two DNA fragments are joined such that the amino acid sequences encoded by the two DNA fragments remain in-frame.

The isolated DNA encoding the VH region can be converted to a full-length heavy chain gene by operatively binding the VH-encoding DNA to another DNA molecule encoding heavy chain constant regions (CH1, CH2 and CH3). The heavy chain constant region may be an IgG1, IgG2, IgG3, IgG4, IgA, IgE, IgM, or IgD constant region.

For a Fab fragment heavy chain gene, the VH-encoding DNA can be operatively linked to another DNA molecule encoding only the heavy chain CH1 constant region.

To create a scFv gene, the VL- and VH-encoding DNA fragments are operatively linked to another fragment encoding a flexible linker, e.g., encoding the amino acid sequence (Gly4-Ser)3, such that the VL and VH sequences can be expressed as a contiguous single-chain protein, with the VL and VH regions joined by the flexible linker.

The nucleic acid sequences of the present invention, such as RNA or DNA, may be isolated from a variety of sources, genetically engineered, amplified, and/or expressed recombinantly. Any recombinant expression system can be used, including, in addition to bacterial, e.g., yeast, insect or mammalian systems. For example, the manipulation of nucleic acids, such as subcloning into expression vectors, labeling probes, sequencing, and hybridization may be performed as known in the art.

Accordingly, the present invention provides a recombinant expression vector including the nucleic acid molecule.

As used herein, the term "vector" refers to a DNA molecule capable of self-replication in prokaryotic and/or eukaryotic cells and is used interchangeably with a recombinant vector, a cloning vector, or an expression vector, which is generally used as a carrier for delivering genes or DNA fragments to cells and the like. The vectors generally include, but are not limited to, an origin of replication that can be replicated in prokaryotic and/or eukaryotic cells, a selection marker gene that can confer resistance to particular conditions/substances such as antibiotic degrading enzymes, a promoter capable of transcription of genes in eukaryotic or prokaryotic cells, and translatable sequences.

One type of vector is a "plasmid," which refers to a circular double stranded DNA loop into which additional DNA segments may be ligated. Another type of vector is a viral vector, in which additional DNA segments may be ligated into the viral genome. Certain vectors are capable of autonomous replication in a host cell into which they are introduced (e.g., bacterial vectors having a bacterial origin of replication and episomal mammalian vectors).

### Preparation of Antibody or Antigen-Binding Fragment thereof

The antibody or antigen-binding fragment thereof of the present invention may be prepared according to conventionally known methods.

In one embodiment, the antibody or antigen binding fragment thereof of the present invention can be prepared using antibody display techniques, such as phage display techniques.

The phage library of the antibody is cloned into a form in which the heavy and light chain variable region genes of the human antibody are fused to the phage surface protein (pill) in a phagemid vector to be expressed in E. coli, and then M13 helper phage is infected to prepare an antibody library in which antibody fragments (scFv or Fab) having sequences of various combinations of heavy and light chain variable regions are displayed on the surface of the phage. From this library, fragments of an antibody binding to a specific antigen are isolated using a panning method, the isolated antibody fragments are characterized, and are then converted into whole IgG form and mass-expressed in animal cells, thereby producing specific human monoclonal antibodies.

As the library, a naive antibody library using an antibody gene already present in the human body may be used, and alternatively, a synthetic antibody library having increased diversity by adding a random synthesis sequence to a CDR of the antibody may be used.

As used herein, the term "phagemid vector" refers to a plasmid DNA having a phage origin of replication, which typically has an antibiotic resistance gene as a selection marker.

A phagemid vector that is used in phage display includes the gIll gene of M13 phage or a portion thereof, and the ScFv gene is ligated to the 5' end of the gIll gene and expressed through a transformant.

A "helper phage" is a phage that provides necessary genetic information so that phagemid is packaged into phage particles. Since phagemid includes the gIII gene of phage or a portion thereof, a host cell (transformant) transformed with the phagemid is infected with the helper phage to provide the remaining phage gene. The helper phages include M13K07 or VCSM13, and mostly include an antibiotic resistance gene such as a kanamycin resistance gene so that a transformant infected with the helper phage can be selected. In addition, the packaging signal is defective, and thus the phagemid gene rather than the helper phage gene is selectively packaged into phage particles.

In another embodiment, an antibody of the invention can be prepared, for example in the form of a monoclonal antibody, by injecting a BCAM antigen into a test subject (e.g., a mouse) according to methods known in the art and then isolating a hybridoma that expresses the antibody having a sequence of interest or functional characteristics.

DNA encoding the monoclonal antibodies is readily isolated and sequenced using conventional procedures (e.g., using oligonucleotide probes that are capable of binding specifically to genes encoding the heavy and light chains of monoclonal antibodies). Hybridoma cells are provided as a preferred source of such DNA. Once isolated, the DNA may be placed into expression vectors, which are then transfected into host cells such as E. coli cells, simian COS cells, Chinese hamster ovary (CHO) cells, or myeloma cells that do not otherwise produce immunoglobulin protein, to obtain the synthesis of monoclonal antibodies in the recombinant host cells.

### Uses and Methods

The antibody or antigen-binding fragment thereof of the present invention specifically binds to BCAM to prevent or treat cancer.

Accordingly, in an embodiment, the present invention provides a method for preventing, ameliorating, or treating cancer, the method comprising administering, to a subject, an effective amount of an anti-BCAM antibody or an antigen-binding fragment thereof.

In another embodiment, the present invention provides a use of an anti-BCAM antibody or an antigen-binding fragment thereof for preventing, ameliorating, or treating cancer.

In another embodiment, the present invention provides a pharmaceutical composition for preventing, ameliorating, or treating cancer, the pharmaceutical composition comprising an anti-BCAM antibody or an antigen-binding fragment thereof. The anti-BCAM antibody or antigen-binding fragment thereof may be included in an effective amount in a pharmaceutical composition.

As used herein, the term "subject" is meant to include both human and non-human animals. Non-human animals include all vertebrates, e.g., mammals and non-mammals, including non-human primates, sheep, dogs, cats, cattle, horses, chickens, amphibians and reptiles, but, for example, non-human primates, sheep, dogs, cats, cattle, and horses are preferred. A preferred subject is a human in need of the prevention or treatment of cancer.

Preferably, the anti-BCAM antibody or antigen-binding fragment thereof of the present invention specifically binds to an BCAM protein thereby effectively binding to cancer cells expressing BCAM, and thus it is possible to inhibit the growth of cancer cells *in vivo* and may be effectively used to prevent, ameliorate, or treat cancer. BCAM is associated with tumor migration, and furthermore, it is known that the overexpression thereof is particularly present in skin cancer, ovarian cancer, pancreatic cancer, breast cancer, and the like (see F.R.M. Latini et al., Blood Cells, Molecules and Diseases 50 (2013) 161-165, and the like). Therefore, the anti-BCAM antibody of the present invention can be used in all cancers in which tumor migration is observed, for example, metastatic cancers, and can also be used in specific carcinomas in which the overexpression thereof is observed.

Examples of cancers of which the growth may be inhibited when the antibody of the present invention is used include, but are not limited to, melanoma (e.g., metastatic malignant melanoma), kidney cancer (e.g., clear cell carcinoma), prostate cancer (e.g., hormone refractory prostate adenocarcinoma), breast cancer, colorectal cancer, rectal cancer, colon cancer, and lung cancer (e.g., non-small cell lung cancer), bone cancer, pancreatic cancer, liver cancer, skin cancer, cancer of the head or neck, uterine cancer, ovarian cancer, rectal cancer, cancer of the anal region, stomach cancer, testicular cancer, carcinoma of the fallopian tubes, carcinoma of the endometrium, carcinoma of the cervix, carcinoma of the vagina, carcinoma of the vulva, Hodgkin's disease, non-Hodgkin's lymphoma, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, chronic or acute leukemia including acute myeloid leukemia, chronic myeloid leukemia, acute lymphoblastic leukemia, chronic lymphocytic leukemia, solid tumors of childhood, lymphocytic lymphoma, bladder cancer, ureteral cancer, carcinoma of the renal pelvis, neoplasm of the central nervous system (CNS), primary CNS lymphoma, tumor angiogenesis, spinal axis tumor, brain stem glioma, pituitary adenoma, Kaposi's sarcoma, epidermoid cancer, squamous cell cancer, T-cell lymphoma, environmentally induced cancers including those induced by asbestos, etc.

In an embodiment, the antibody or antigen-binding fragment thereof of the present invention can be used to prevent or treat cancer by specifically binding to the BCAM protein to form an antibody-drug conjugate (ADC). Accordingly, in an embodiment, the present invention provides an anti-BCAM antibody or antigen-binding fragment in the form conjugated with a drug. In an embodiment, the drug includes, but is not limited to, a cytotoxic agent (e.g., a chemotherapeutic agent), a prodrug converting enzyme, a radioactive isotope or compound, or a toxin. Drugs and linkers that may be included in the ADC and preparation methods thereof may be according to methods known in the art.

The antibody or antigen-binding fragment of the present invention may be used alone or in combination with other anticancer therapies. Other anticancer therapies may include, for example, standard cancer therapies (e.g., chemotherapies, radiotherapies, or surgery); or other anticancer agents, such as cytotoxic, cytostatic, antihormone, anti angiogenic or antimetabolite agents, targeted agents, immune stimulating or immune modulating agents, immune checkpoint inhibitors, or antibodies conjugated with cytotoxic agents, cytostatic, and other toxic agents.

Preferably, the antibody or antigen-binding fragment of the present invention may be used in combination with other anticancer agents such as immune checkpoint inhibitors, chemotherapeutic agents, radiotherapies, or the like.

The immune checkpoint inhibitors may be, for example, anti-CTLA-4 antibodies (e.g., ipilimumab), anti-PD-1 antibodies (e.g., pembrolizumab, nivolumab), or anti-PD-L1 antibodies (e.g., atezolizumab, avelumab, durvalumab). The chemotherapeutic agents include, but are not limited to, alkylating agents, antimetabolites, kinase inhibitors, spindle poison plant alkaloids, cytotoxic/antitumor antibiotics, topoisomerase inhibitors, photosensitizers, anti-estrogens and selective estrogen receptor modulators (SERMs), anti-progesterones, estrogen receptor down-regulators (ERDs), estrogen receptor antagonists, luteinizing hormone-releasing hormone agonists, anti-androgens, aromatase inhibitors, EGFR inhibitors, VEGF inhibitors, anti-sense oligonucleotides that inhibit expression of genes implicated in abnormal cell proliferation or tumor growth. Specific examples of chemotherapeutic agents of the present invention include gemcitabine, vinorelbine, etoposide (VP-16), platinum analogs such as cisplatin or carboplatin, taxoids such as paclitaxel, albumin-binding paclitaxel, and doxetaxel, or the like.

When the antibody or antigen-binding fragment of the present invention is used in combination with other anticancer agents, these may be administered separately or may be applied in the form of a combination product in which a plurality of active ingredients are present in a single pharmaceutical formulation. When these are administered as separate formulations, the two formulations may be administered sequentially or simultaneously. For simultaneous administration, these are given together to the patient. For sequential administration, these may be given at a time interval that is not long, for example, these formulations may be administered to a patient within a period of 12 hours or less, or 6 hours or less.

In an embodiment, the present invention provides a method of preventing, ameliorating, or treating cancer, the method comprising administering, to a subject, an effective amount of an antibody or an antigen-binding fragment thereof in combination with an additional anticancer agent. The embodiment includes not only simultaneously administering a single composition containing an anti-BCAM antibody or an antigen-binding fragment together with an additional anticancer agent to a patient in need thereof, but also simultaneously or sequentially administering compositions separately and respectively containing the anti-BCAM antibody or antigen-binding fragment and the additional anticancer agent to a patient in need thereof.

In another embodiment, the present invention provides a use of an anti-BCAM antibody or antigen-binding fragment thereof for use in combination with an additional anticancer agent for preventing, ameliorating or treating cancer.

In another embodiment, the present invention provides a pharmaceutical composition or combination for preventing, ameliorating, or treating cancer, the composition or combination comprising an anti-BCAM antibody or an antigen-binding fragment thereof, and an additional anticancer agent. The pharmaceutical composition or combination herein comprising an anti-BCAM antibody or an antigen-binding fragment thereof, and an additional anticancer agent, include not only the case where the two components are physically present together in the form of a single formulation, but also the case where the two components are administered simultaneously or sequentially in separate formulations, wherein the two drugs may be provided separately or together in a single kit. Accordingly, the present invention provides a kit for preventing, ameliorating, or treating cancer, the kit comprising an anti-BCAM antibody or an antigen-binding fragment thereof and an additional anticancer agent.

### Pharmaceutical Composition

The present invention provides a pharmaceutical composition comprising an anti-BCAM antibody or an antigen-binding fragment thereof. The composition may contain inactive ingredients, that is, pharmaceutically acceptable excipients (see Handbook of Pharmaceutical Excipients, etc.). Compositions of therapeutic and diagnostic formulations may be prepared in the form of, for example, freeze-dried powder, slurry, aqueous solution or suspension by mixing the formulations with physiologically acceptable carriers, excipients, or stabilizers.

Suitable route of administration includes parenteral administration, such as intramuscular, intravenous, or subcutaneous administration. Administration of the antibodies used in the pharmaceutical composition of the present invention or used to practice the methods of the present invention may be carried out by a variety of conventional methods such as topical application, or intradermal, subcutaneous, intraperitoneal, parenteral, intraarterial, or intravenous injection. In an embodiment, the antibody of the present invention is administered intravenously or subcutaneously.

### Modes for the Invention

Hereinafter, the present invention will be described in more detail with reference to Examples. It will be apparent to those skilled in the art that these Examples are only intended to describe the present invention in more detail, and the scope of the present invention is not limited by these Examples according to the subject matter of the present invention.

### Example 1: Preparation of BCAM-specific Antibody through Phage Display

### 1.1 Preparation and Selection of scFv Antibody Library through Phage Display

The antibody of the present invention was prepared through a phage display method.

mRNA of the variable regions of the heavy and light chains of the antibody obtained from human blood or bone marrow was amplified by PCR to synthesize cDNA. Cloning was performed on phagemid vector using restriction enzymes and then expression was carried out by electroporation on *E. coli.* Subsequently, infection was performed with helper phage to prepare a human library in the form of scFv.

The library was screened for an antibody that binds with high affinity to the target antigen BCAM through biopanning. A positive clone binding to BCAM was selected, and a unique scFv sequence was selected for BCAM through sequencing.

### 1.2 Preparation of Anti-BCAM IgG4 Antibodies 111a to 111c

For the production of antibodies in the form of human IgG4, the light chain and heavy chain regions of scFv selected in Example 1.1 were cloned to an expression vector. DNA was transient transfected and cultured to express antibodies and the culture medium was bound to kappa region capture to elute the antibodies to prepare three anti-BCAM human IgG4 antibodies (111a, 111b, and 111c).

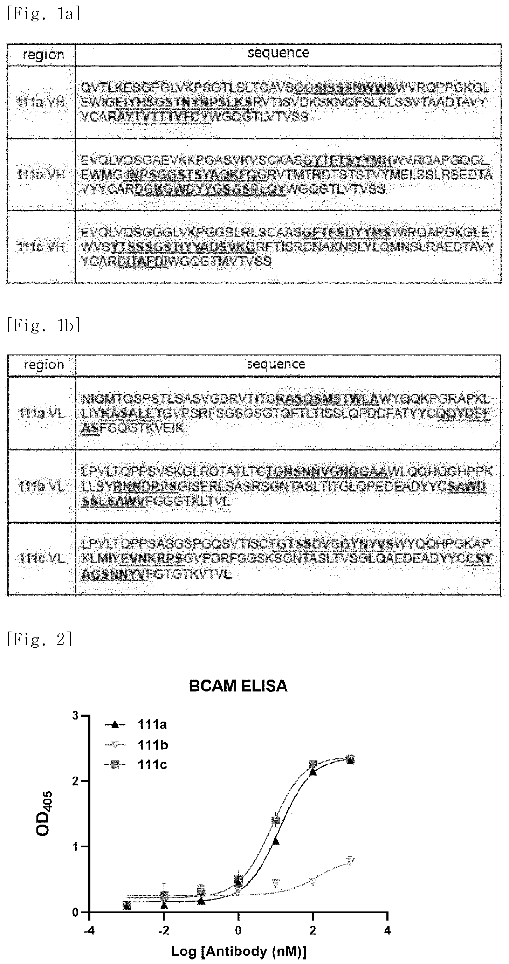

Amino acid sequences of the light and heavy chain variable regions of each IgG4 antibody, and amino acid sequences of the CDRs of each variable region are each shown in FIGS. 1a and 1b, and Tables 1 and 2 (the bold and underlined sequences in FIGS. 1a and 1b and Table 1 represent each CDR).

**[Table 1]**

| SEQ ID No | Region | Sequence |
|---|---|---|
| SEQ ID No. 1 | 111a heavy chain variable region (VH) | QVTLKESGPGLVKPSGTLSLTCAVSGGSISSSNWWSW VRQPPGKGLEWIGEIYHSGSTNYNPSLKSRVTISVDKS KNQFSLKLSSVTAADTAVYYCARAYTVTTTYFDYWG QGTLVTVSS |
| SEQ ID No. 2 | 111a light chain variable region (VL) | NIQMTQSPSTLSASVGDRVTITCRASQSMSTWLAWYQ QKPGRAPKLLIYKASALETGVPSRFSGSGSGTQFTLTIS SLQPDDFATYYCQQYDEFASFGQGTKVEIK |
| SEQ ID No. 3 | 111b heavy chain variable region (VH) | EVQLVQSGAEVKKPGASVKVSCKASGYTFTSYYMHW VRQAPGQGLEWMGIINPSGGSTSYAQKFQGRVTMTR DTSTSTVYMELSSLRSEDTAVYYCARDGKGWDYYGS GSPLQYWGQGTLVTVSS |
| SEQ ID No. 4 | 111b light chain variable region (VL) | LPVLTQPPSVSKGLRQTATLTCTGNSNNVGNQGAAWL QQHQGHPPKLLSYRNNDRPSGISERLSASRSGNTASLTI TGLQPEDEADYYCSAWDSSLSAWVFGGGTKLTVL |
| SEQ ID No. 5 | 111c heavy chain variable region (VH) | EVQLVQSGGGLVKPGGSLRLSCAASGFTFSDYYMSWI RQAPGKGLEWVSYTSSSGSTIYYADSVKGRFTISRDNA KNSLYLQMNSLRAEDTAVYYCARDITAFDIWGQGTM VTVSS |
| SEQ ID No. 6 | 111c light chain variable region (VL) | LPVLTQPPSASGSPGQSVTISCTGTSSDVGGYNYVSWY QQHPGKAPKLMIYEVNKRPSGVPDRFSGSKSGNTASLT VSGLQAEDEADYYCCSYAGSNNYVFGTGTKVTVL |

**[Table 2]**

| SEQ ID No | Region | Sequence |
|---|---|---|
| SEQ ID No: 7 | 111a VH CDR1 | GGSISSSNWWS |
| SEQ ID No: 8 | 111a VL CDR1 | RASQSMSTWLA |
| SEQ ID No: 9 | 111b VH CDR1 | GYTFTSYYMH |
| SEQ ID No: 10 | 111b VL CDR1 | TGNSNNVGNQGAA |
| SEQ ID No: 11 | 111c VH CDR1 | GFTFSDYYMS |
| SEQ ID No: 12 | 111c VL CDR1 | TGTSSDVGGYNYVS |
| SEQ ID No: 13 | 111a VH CDR2 | EIYHSGSTNYNPSLKS |
| SEQ ID No: 14 | 111a VL CDR2 | KASALET |
| SEQ ID No: 15 | 111b VH CDR2 | IINPSGGSTSYAQKFQG |
| SEQ ID No: 16 | 111b VL CDR2 | RNNDRPS |
| SEQ ID No: 17 | 111c VH CDR2 | YTSSSGSTIYYADSVKG |
| SEQ ID No: 18 | 111c VL CDR2 | EVNKRPS |
| SEQ ID No: 19 | 111a VH CDR3 | AYTVTTTYFDY |
| SEQ ID No: 20 | 111a VL CDR3 | QQYDEFAS |
| SEQ ID No: 21 | 111b VH CDR3 | DGKGWDYYGSGSPLQY |
| SEQ ID No: 22 | 111b VL CDR3 | SAWDSSLSAWV |
| SEQ ID No: 23 | 111c VH CDR3 | DITAFDI |
| SEQ ID No: 24 | 111c VL CDR3 | CSYAGSNNYV |

### Example 2: Confirmation of Avidity of Anti-BCAM IgG4 Antibody

### 2.1 Confirmation of Binding Ability to Antigenic Protein according to ELISA

In order to confirm the binding ability of each antibody prepared in Example 1 to an antigen protein (BCAM protein), an ELISA (Enzyme-Linked Immunosorbent Assay) test was performed. The antigenic protein was diluted in PBS buffer and prepared to a concentration of 15 nM. Each well of a 96-well half plate (Costar, Cat. No. 3690) was coated with 50 µL of the antigenic protein, followed by overnight incubation at 4 °C. Next day, after all the solutions in the plate were removed, a blocking buffer (3% BSA in PBS) was added to each well, followed by incubation at 37 °C for 1 hour. Three antibody samples (111a, 111b, and 11 1c) were sequentially diluted in the blocking buffer from 1 µM to 7 points (1 pM) at a dilution ratio of 10-fold. After the blocking process was completed, the buffer was removed from the well, and the antibodies prepared by the dilution was added to each well by 50 µL, followed by incubation at 37 °C for 2 hours. The incubated well was washed with 0.1% PBST (0.1% Tween 20 in PBS) and were treated with HRP-conjugated anti-human IgG Fc antibodies (ThermoFisher Scientific, Cat. No.: 31423) which were secondary antibodies for antibody detection at 37 °C for 1 hour, and then washed again with 0.1% PBST. ABTS solution (ThermoFisher Scientific, Cat. No.: 00-2024) was added to each well by 50 µL for coloring, and reacted at room temperature for 10 minutes, and an optical density at 405 nm (OD₄₀₅) was measured. The OD₄₀₅ values according to the concentration of antibodies are shown in FIG. 2 and Table 3. In order to compare the degree of avidity of each antibody to antigens, the EC50 concentration of each antibody was calculated, and the results are shown in Table 4.

As a result, it was confirmed that the antibodies 111a, 111b, and 111c tested through ELISA experiment had an excellent binding ability to BCAM.

**[Table 3]**

| Abs (nM) | 111a | 111b | 111c |
|---|---|---|---|
| 1000 | 2.32 ± 0.04 | 0.76 ± 0.09 | 2.34 ± 0.06 |
| 100 | 2.14 ± 0.03 | 0.46 ± 0.03 | 2.26 ± 0.01 |
| 10 | 1.09 ± 0.03 | 0.43 ± 0.06 | 1.41 ± 0.12 |
| 1 | 0.46 ± 0.06 | 0.33 ± 0.06 | 0.50 ± 0.15 |
| 0.1 | 0.18 ± 0.06 | 0.35 ± 0.07 | 0.31 ± 0.09 |
| 0.01 | 0.11 ± 0.01 | 0.15 ± 0.00 | 0.25 ± 0.19 |
| 0.001 | 0.10 ± 0.01 | 0.10 ± 0.00 | 0.11 ± 0.01 |

**[Table 4]**

| Antibody | 111a | 111b | 111c |
|---|---|---|---|
| EC50 (nM) | 12.5 | 126.9 | 7.7 |

### 2.2 Confirmation of Binding Ability to Antigenic Protein according to Fluorescence Activated Cell Sorting (FACS, Flow Cytometry)

A FACS test was performed on the three antibodies prepared in Example 1.

Human BCAM antigens were expressed in HEK293FT cells via transfection (using 293FTBCAM1 plasmid). The transfected HEK293FT cells (1×10⁵ cells) were suspended in PBS and 50 µL thereof was seeded on a plate.

Separately, each of the anti-BCAM antibodies of Example 1 and isotype control (human IgG) antibodies was initially diluted at a concentration of 45 µg/mL using PBS and diluted 12 times through 3-fold dilution, and the plate was incubated at an appropriate time and at an appropriate temperature. As a negative control, PBS buffer was used.

Then, after washing, AlexaFluor^{®} 647 AffiniPure Goat Anti-Human IgG (H+L) (min X Bov, Hrs, Ms Sr Prot) (Jackson) (1 µg/mL) was added thereto and the plate was incubated again, washed again with PBS, and the binding degree of antigen-antibody was analyzed with a flow cytometer.

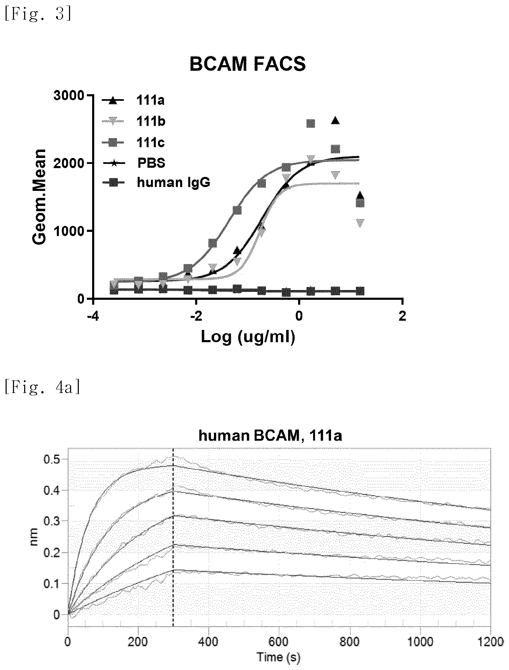

The results are shown in Table 5 and FIG. 3.

**[Table 5]**

| | **111a** | **111b** | **111c** |
|---|---|---|---|
| EC50 (µg/ml) | 0.1885 | 0.1709 | 0.0427 |

### 2.3 Confirmation of Binding Ability to Antigenic Protein according to Bio-layer Interferometry (BLI)

The binding ability confirmation test according to BLI for the three antibodies prepared in Example 1 was performed using Octet (Octet QK, ForteBio).

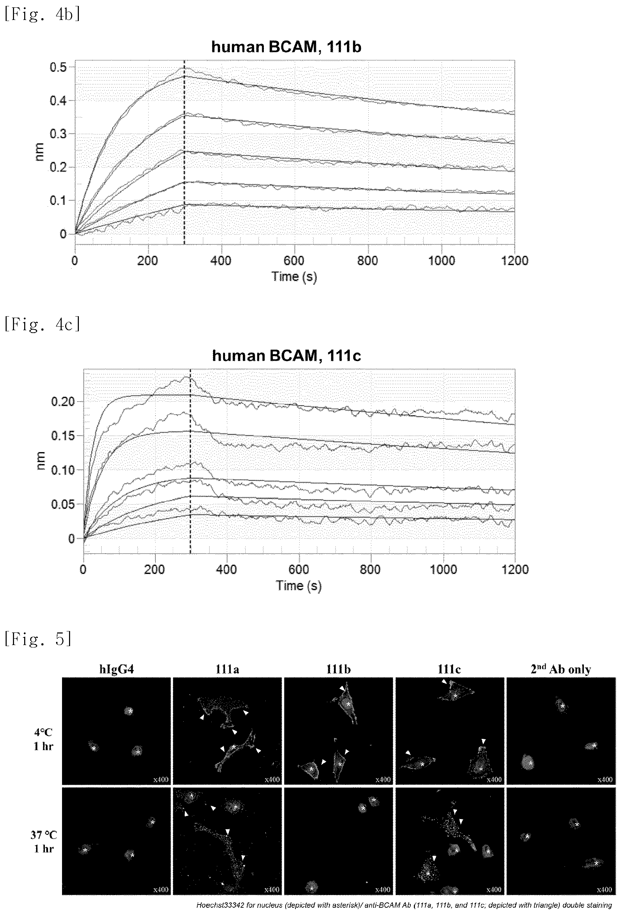

Specifically, in order to bind human BCAM (antigen) using amine coupling, Reactive 2nd Generation (AR2G) biosensors (Cat. No. 18-5094, Sartorius) were mounted and activated using the AE2G reagent kit (Cat. No. 18-5095). Thereafter, the biosensors were put in an antigen solution for 10 minutes to bind the human BCAM, and put in an ethanolamine solution for 5 minutes to terminate the reaction. The biosensors to which the human BCAM was bound was put into a buffer containing the prepared three different antibodies 111a, 111b, and 111c, and the binding reaction was monitored for 5 minutes, followed by a dissociation reaction for 15 minutes. At this time, antibody solutions were sequentially diluted, and association and dissociation rates depending on different concentrations were verified. The association (Ka) and dissociation (Kd) rate constants were determined by fitting them to a 1:1 binding model using a curve-fitting software. The binding dissociation equilibrium constant (KD) was calculated with KD = Kd/Ka. The binding dynamics data are shown in Table 6 below, and the binding sensor-gram is shown in FIGS. 4a, 4b and 4c.

**[Table 6]**

| Analyte | Ligand | ka (1/Ms) | kd (1/s) | K_{D} (M) |
|---|---|---|---|---|
| 111a | HumanBCAM | 1.61 X 10³ | 3.97 X 10⁻⁴ | 2.47 X 10⁻⁹ |
| 111b | | 2.34 X 10⁴ | 3.06 X 10⁻⁴ | 1.31 X 10⁻⁸ |
| 111c | | 3.04 X 10⁶ | 2.55 X 10⁻⁴ | 8.40 X 10⁻¹¹ |

From this, it was confirmed that the antibody of the present invention binds to the human BCAM protein with high affinity.

### Example 3: Confirmation of Cellular Internalization of Antibodies through Confocal Laser Scanning Microscopy

An experiment was performed to confirm the cellular internalization of the antibodies using the three antibodies prepared in Example 1.

5 × 10⁴ MKN-1 (human stomach cancer cell line, KCLB) cells were plated in a cell culture slide (SPL, Korea). Next day, it was confirmed that the cells were attached to plate, and after removing the existing cell culture medium, the antibodies 111a, 111b, and 111c prepared in Example 1 were diluted in PBS to prepare 10 µg/mL or 40 µg/mL, and then each well was treated with 500 µL of the antibodies. After the antibodies were reacted at 4 °C for 1 hour, the remaining amount of the antibodies was removed, and the cell slide was washed three times with PBS. Cell slides were divided into two groups, and one group was used as a negative control for the cellular internalization and the other group was used as a cellular internalization inducing group. The cell slide to be used as the negative control was fixed (treated at room temperature for 10 minutes) using 4% paraformaldehyde (PFA, Sigma, USA), and the cell slide to be used as the cellular internalization inducing group was transferred to a 37 °C incubator and incubated for 1 hour, and then fixed using 4% PFA as in the negative control. Each cell slide, which had been fixed, was washed with PBS to completely remove PFA, then treated with 0.1% TritonX100 at room temperature for 15 minutes, and prepared to allow the permeabilization of secondary antibodies. After the cell slide was subjected to PBS washing and blocking processes (treated with 3% BSA at room temperature for 20 minutes), the cell slide was treated with the secondary antibodies conjugated with fluorescence (10 µ g/mL Alexa488-conjugated Goat anti-human IgG; Invitrogen, USA) at 4 °C for 1 hour. The cell slides that had been subjected to the second antibody treatment were each treated with Hoechst 33342 (Invitrogen, USA) at room temperature for 3 minutes for nuclear staining, washed with PBS, treated with a mounting solution (Agilent Technologies, USA), and covered with a cover glass to complete a slide sample. The degree of the cellular internalization of antibodies was observed under 400x magnification using the LSM 710 confocal laser scanning microscopy (Carl Zeiss, Germany).

The results are shown in FIG. 5. In the case of the negative control group (4 °C and 1 hr antibody treatment condition that did not induce the cellular internalization, top photograph of FIG. 5), the three antibodies were each bound to the surface of the cancer cell, but in the case of the cellular internalization induction condition, 37 °C and 1 hr antibody treatment condition (bottom photograph of FIG. 5), it was confirmed that each antibody was internalized into the cell so that the fluorescent signal on the surface of the cell was reduced while the fluorescent signal inside the cell increased (the fluorescent signal of the nucleus-stained portion is indicated by an asterisk, and the fluorescent signal of each antibody-stained portion is indicated by a triangle). These results show that the antibody of the present invention has the potential to deliver a cytotoxic drug into the cell through the cellular internalization, that is, the antibody may be used in the form of an ADC conjugated with a drug in the future.

## Claims

1. An anti-BCAM antibody or an antigen binding fragment thereof comprising:
a heavy chain CDR1 comprising an amino acid sequence of any one selected from the group consisting of SEQ ID NOs: 7, 9 and 11;
a heavy chain CDR2 comprising an amino acid sequence of any one selected from the group consisting of SEQ ID NOs: 13, 15 and 17;
a heavy chain CDR3 comprising an amino acid sequence of any one selected from the group consisting of SEQ ID NOs: 19, 21 and 23;
a light chain CDR1 comprising an amino acid sequence of any one selected from the group consisting of SEQ ID NOs: 8, 10 and 12;
a light chain CDR2 comprising an amino acid sequence of any one selected from the group consisting of SEQ ID NOs: 14, 16 and 18; and
a light chain CDR3 comprising an amino acid sequence of any one selected from the group consisting of SEQ ID NOs: 20, 22 and 24.

2. The anti-BCAM antibody or antigen binding fragment thereof of claim 1, comprising:
(a) the heavy chain CDR1 comprising the amino acid sequence of SEQ ID NO: 7;
the heavy chain CDR2 comprising the amino acid sequence of SEQ ID NO: 13;
the heavy chain CDR3 comprising the amino acid sequence of SEQ ID NO: 19;
the light chain CDR1 comprising the amino acid sequence of SEQ ID NO: 8;
the light chain CDR2 comprising the amino acid sequence of SEQ ID NO: 14; and
the light chain CDR3 comprising the amino acid sequence of SEQ ID NO: 20;
(b) the heavy chain CDR1 comprising the amino acid sequence of SEQ ID NO: 9;
the heavy chain CDR2 comprising the amino acid sequence of SEQ ID NO: 15;
the heavy chain CDR3 comprising the amino acid sequence of SEQ ID NO: 21;
the light chain CDR1 comprising the amino acid sequence of SEQ ID NO: 10;
the light chain CDR2 comprising the amino acid sequence of SEQ ID NO: 16; and
the light chain CDR3 comprising the amino acid sequence of SEQ ID NO: 22; or
(c) the heavy chain CDR3 comprising the amino acid sequence of SEQ ID NO: 11;
the heavy chain CDR2 comprising the amino acid sequence of SEQ ID NO: 17; the heavy chain CDR3 comprising the amino acid sequence of SEQ ID NO: 23;
the light chain CDR1 comprising the amino acid sequence of SEQ ID NO: 12;
the light chain CDR2 comprising the amino acid sequence of SEQ ID NO: 18; and
the light chain CDR3 comprising the amino acid sequence of SEQ ID NO: 24.

3. The anti-BCAM antibody or antigen binding fragment thereof of claim 1,
comprising: a heavy chain variable region of any one selected from the group consisting of SEQ ID NOs: 1, 3 and 5; and
a light chain variable region of any one selected from the group consisting of SEQ ID NOs: 2, 4 and 6.

4. The anti-BCAM antibody or antigen binding fragment thereof of claim 1, comprising:
(a) a heavy chain variable region comprising an amino acid sequence of SEQ ID NO: 1, and a light chain variable region comprising an amino acid sequence of SEQ ID NO: 2;
(b) a heavy chain variable region comprising an amino acid sequence of SEQ ID NO: 3, and a light chain variable region comprising an amino acid sequence of SEQ ID NO: 4; or
(c) a heavy chain variable region comprising an amino acid sequence of SEQ ID NO: 5, and a light chain variable region comprising an amino acid sequence of SEQ ID NO: 6.

5. The anti-BCAM antibody or antigen binding fragment thereof of claim 1, wherein the anti-BCAM antibody is a multi-specific antibody.

6. The anti-BCAM antibody or antigen binding fragment thereof of claim 1, wherein the antigen-binding fragment is Fab, Fab', Fab'-SH, Fv, a single chain antibody scFv, a F(ab')2 fragment, VL, VH, a diabody, a triabody, a tetrabody, a minibody (scFV(CH3)2), IgG deltaCH2, scFv-Fc, (scFv)2-Fc, Fynomer, dual-affinity re-targeting (DART), or TRIDENT.

7. The anti-BCAM antibody or antigen-binding fragment thereof of claim 1, binding to a human BCAM protein with a binding dissociation equilibrium constant (K_{D}) of 1 × 10⁻⁷ M or less.

8. The anti-BCAM antibody or antigen-binding fragment thereof of claim 1, wherein the antibody is a chimeric antibody, a humanized antibody, or a human antibody.

9. An antibody-drug conjugate comprising the anti-BCAM antibody or antigen-binding thereof of any one of claims 1 to 8.

10. A nucleic acid molecule encoding the anti-BCAM antibody or antigen-binding fragment thereof of any one of claims 1 to 8.

11. A recombinant expression vector comprising the nucleic acid molecule of claim 10.

12. A pharmaceutical composition for preventing or treating cancer, the pharmaceutical composition comprising the anti-BCAM antibody or antigen-binding thereof of any one of claims 1 to 8.

13. The pharmaceutical composition of claim 12, further comprising an additional anticancer agent.

14. The pharmaceutical composition of claim 13, wherein the anti-BCAM antibody or antigen-binding fragment thereof, and the additional anticancer agent is simultaneously administered as a single formulation, or is simultaneously or sequentially administered as separate formulations.

15. An anti-BCAM antibody or antigen-binding fragment thereof that specifically binds to human BCAM, which binds to the same epitope as an epitope to which the antibody 111a, 111b or 111c binds.
